# EUROPEAN PATENT SPECIFICATION

(11) **EP 4 360 603 B1**
(45) Date of publication and mention of the grant of the patent: **06.08.2025**
(21) Application number: 22858157.5
(22) Date of filing: 17.06.2022
(51) Int. Cl.: A61F 13/49, A61F 13/56

(54) **DISPOSABLE WEARABLE ARTICLE**
WEARABLE-EINWEGARTIKEL
ARTICLE JETABLE POUVANT ÊTRE PORTÉ

(30) Priority: 18.08.2021 JP 2021133594
(43) Date of publication of application: 01.05.2024
(73) Proprietor: Zuiko Corporation, Ibaraki-shi Osaka 5670082 (JP)
(72) Inventor: SATO Hitoshi, Ibaraki-shi, Osaka 567-0082 (JP); FUKUHARA Takeshi, Ibaraki-shi, Osaka 567-0082 (JP); NAGATA Kazuma, Ibaraki-shi, Osaka 567-0082 (JP)
(74) Representative: Hasegawa, Kan
(86) International application number: PCT/JP2022/024339
(87) International publication number: WO 2023/021833

(56) References cited:
- EP-B1- 2 240 144
- JP-A- 2008 272 250
- JP-A- 2016 013 470
- US-A- 6 069 097
- US-A1- 2013 211 363
- US-A1- 2014 259 579

## Description

### TECHNICAL FIELD

The present invention relates to a disposable wearable article, and more particularly, it relates to a disposable wearable article provided with a fastener member.

### BACKGROUND ART

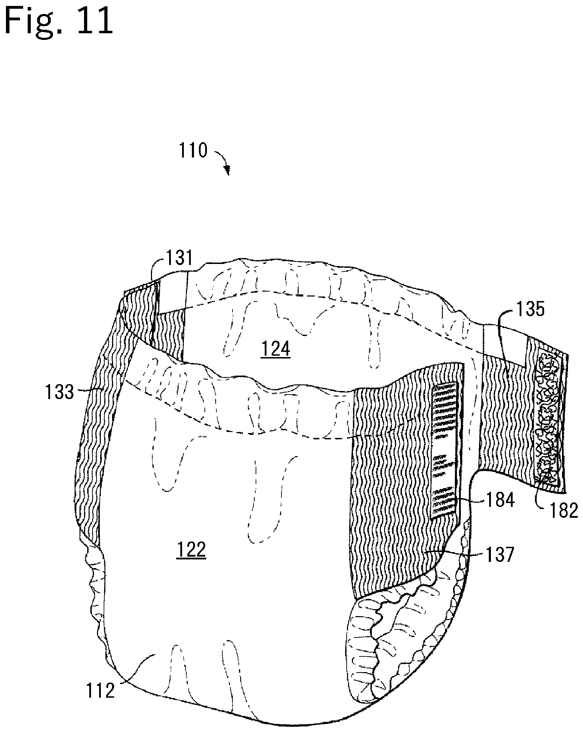

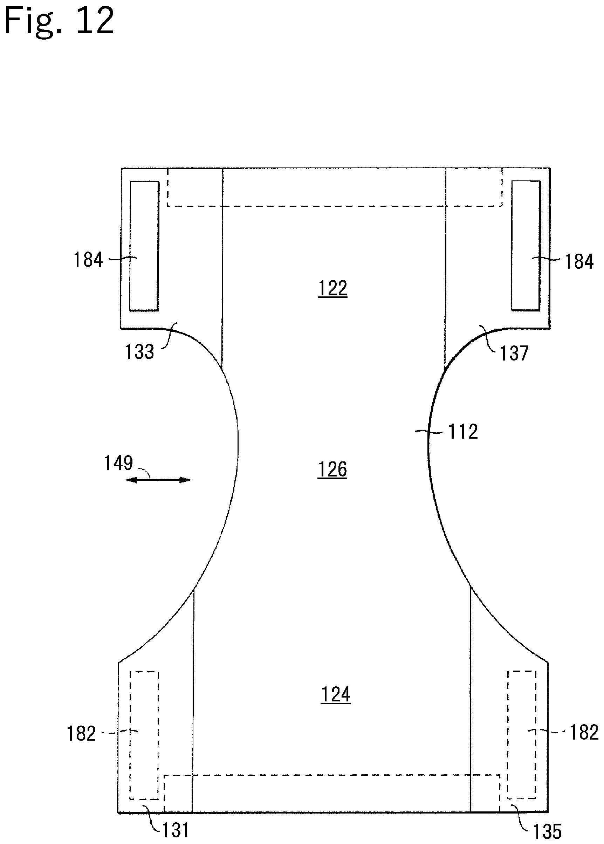

It is known to use fastener members in disposable wearable articles such as paper diapers and adult incontinence products. For example, Fig. 11 is a perspective view of such a disposable wearable article 110, and Fig. 12 is a plan view for illustrating the outer side thereof in a developed state. As illustrated in Figs. 11 and 12, flap parts 131, 133, 135, and 137 extend from a front part 122 and a back part 124 to both sides of a crotch part 126 of an absorptive main body 112 in a around-torso direction illustrated with an arrow 149. In the flap parts 131, 133, 135, and 137, fastener members 182 and 184 are disposed. For example, one fastener member 182 is a loop fastener, the other fastener member 184 is a hook fastener, and these fastener members 182 and 184 are overlaid with each other for fastening.

The fastener members 182 and 184 are disposed at a distance from tip ends in the around-torso direction of the flap parts 131, 133, 135, and 137. Thus, hard fastening surfaces of the fastener members 182 and 184 can be prevented from being touched when the fastener members 182 and 184 are fastened. Besides, the fastening between the fastener members 182 and 184 can be released by picking up portions, which protrude to the ends on the tip side in the around-torso direction beyond the fastener members 182, of the flap parts 131 and 135 extending from the back part 124 (see, for example, Patent Literature 1).

### CITATION LIST

### PATENT LITERATURE

[Patent Literature 1] Japanese Patent No. 5411158

### Summary of Invention

### PROBLEM TO BE SOLVED BY THE INVENTION

In such a configuration, when the flap part is made of a nonwoven fabric, a portion on the tip side in the around-torso direction of the flap part disposed beyond the fastener member may be folded to attach to the fastening surface of the fastener member, and thus, a part of the fastening surface of the fastener member may be covered. When the fastener members are fastened in this state, the fastening area is insufficient, which causes a problem of weak fastening power. In a hook fastener in particular, since a large number of hook portions are disposed therein, the nonwoven fabric of the tip end of the flap part may be caught by the hook portions in some cases.

In consideration of these circumstances, a problem to be solved by the present invention is to provide a disposable wearable article that can ensure sufficient fastening power of a fastener member by suppressing folding of a portion protruding to the end on the tip side of a flap part beyond the fastener member of the flap part, and preventing the flap part from covering the fastener member.

### MEANS FOR SOLVING THE PROBLEM

The present invention provides a disposable wearable article having the following configuration for solving the above-described problem.

The disposable wearable article includes (a) an absorptive main body in which an absorber is disposed between a top sheet and a back sheet; (b) a pair of flap parts that are provided in one of a front part and a back part disposed on both side of a crotch part of the absorptive main body, and extend to both sides in a around-torso direction; and (c) a pair of fastener members that are respectively provided in the pair of flap parts at a distance from tip ends in the around-torso direction of the flap parts to be attachably and detachably joined to an opposing part provided in the absorptive main body. Each of the flap parts is made of a thermoplastic nonwoven fabric, and includes, in an extending part between the tip end of the flap part and the fastener member, a thermally-fused part having bending rigidity of the nonwoven fabric increased by thermally fusing the nonwoven fabric.

According to the above-described configuration, each flap part includes a thermally-fused part in a portion protruding to the end on the tip side in the around-torso direction beyond the fastener member, namely, the extending part. The thermally-fused part suppresses folding of the end on the tip side of the flap part. Thus, the end on the tip side in the around-torso direction (hereinafter, simply referred to as the "end on the tip side") of the flap part is prevented from covering a fastening surface of the fastener member, and thus, sufficient fastening power of the fastener member can be ensured.

The thermally-fused part may be formed in a whole region in the around-torso direction of the extending part, or may be formed in a partial region in the around-torso direction of the extending part.

Preferably, the thermally-fused part continuously extends from the extending part into a region of the flap part where the fastener member is disposed.

In this case, folding of the end on the tip side of the flap part beyond the fastener member can be suppressed at the base on the side of the fastener member. As a result, sufficient fastening power of the fastener member can be more definitely ensured.

Preferably, each of the flap parts includes the thermally-fused part in a plural number linearly extending at a right angle to the tip end of the flap part.

In this case, the thermally-fused part can be easily formed, and the disposable wearable article can be easily mass-produced.

Preferably, each of the flap parts includes the thermally-fused part formed in a lattice shape.

In this case, folding in all directions of the end on the tip side of the flap part is suppressed. As a result, sufficient fastening power of the fastener member can be more definitely ensured.

Preferably, each of the flap parts includes the thermally-fused part formed adjacent to a whole region between a first end and a second end, in a direction crossing the around-torso direction at a right angle, of the tip end of the flap part.

In this case, folding of the end on the tip side of the flap part is suppressed in the whole region along the tip end of the flap part. As a result, sufficient fastening power of the fastener member can be more definitely ensured.

### EFFECTS OF THE INVENTION

A disposable wearable article of the present invention can suppress folding of a portion of a flap part protruding to the end on the tip side in a around-torso direction beyond a fastener member (extending part) to prevent the flap part from covering the fastener member, and thus, sufficient fastening power of the fastener member can be ensured.

### BRIEF DESCRIPTION OF THE DRAWINGS

[Fig. 1] Fig. 1 is a plan view of a disposable wearable article. (Example 1)
[Fig. 2] Fig. 2 is an enlarged view of a main part of Fig. 1. (Example 1)
[Fig. 3] Fig. 3 is an explanatory diagram of production processes of the disposable wearable article. (Example 1)
[Fig. 4] Fig. 4 is an explanatory diagram of production processes of the disposable wearable article. (Example 1)
[Fig. 5] Fig. 5 is an enlarged view of the vicinity of a tip end planned region. (Example 1)
[Fig. 6] Fig. 6 is an enlarged view of the vicinity of a tip end planned region. (Modification Example 1)
[Fig. 7] Fig. 7 is an enlarged view of the vicinity of a tip end planned region. (Modification Example 2)
[Fig. 8] Fig. 8 is an enlarged view of the vicinity of a tip end planned region. (Modification Example 3)
[Fig. 9] Fig. 9 is an enlarged view of the vicinity of a tip end planned region. (Modification Example 4)
[Fig. 10] Fig. 10 is a plan view of a disposable wearable article. (Example 2)
[Fig. 11] Fig. 11 is a perspective view of a disposable wearable article. (Conventional Example 1)
[Fig. 12] Fig. 12 is a plan view of the disposable wearable article. (Conventional Example 1)

### MODE FOR CARRYING OUT THE INVENTION

Preferable embodiments of the present invention will now be described with reference to the accompanying drawings.

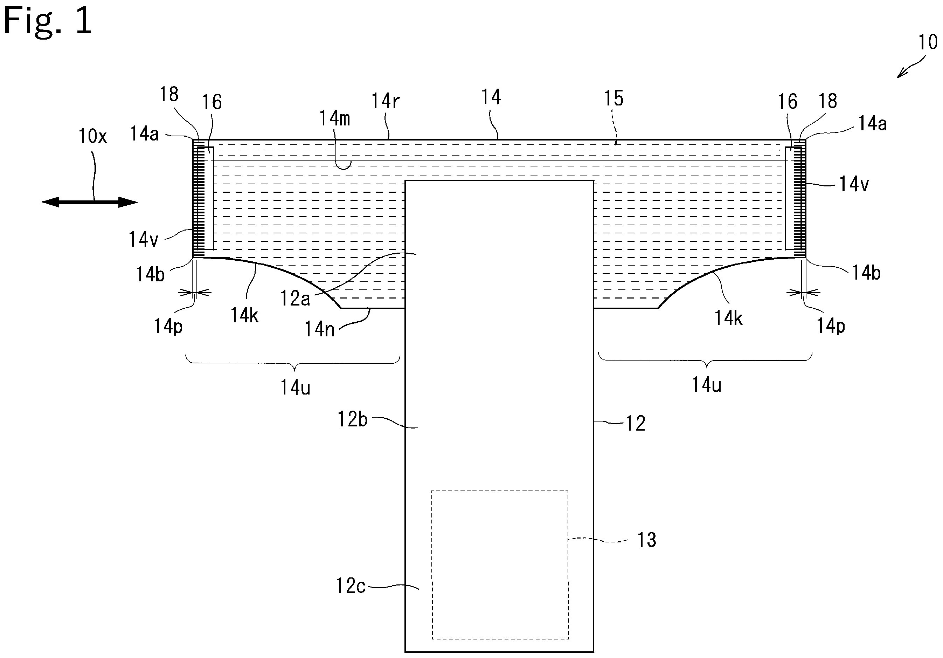

### <Example 1> A disposable wearable article 10 of Example 1 will now be described with reference to Figs. 1 to 5.

Fig. 1 is a plan view of the disposable wearable article 10, and illustrates the skin surface side thereof in a developed state. As illustrated in Fig. 1, in the disposable wearable article 10, an absorptive main body 12 is joined to a flap member 14.

Although not illustrated in the drawings, in the absorptive main body 12, an absorber for absorbing a liquid is disposed between a liquid permeable top sheet and a liquid impermeable back sheet. The absorber is disposed centering around a crotch part 12b of the absorptive main body 12 to extend into a back part 12a and a front part 12c on both sides of the crotch part.

In the absorptive main body 12, an opposing member 13 is provided on the other side of the top sheet of the absorptive main body 12, namely, on the non-skin surface side. The opposing member 13 may be provided in one portion, or in a plurality of portions. The opposing member 13 covers a part or the whole of the non-skin surface side of the absorptive main body 12, and is exposed to the non-skin surface side. The opposing member 13 forms an opposing part to which a fastener member 16 described below is attachably and detachably joined, and may be, for example, a loop fastener or the like, or a nonwoven fabric sheet or the like.

In the flap member 14, a stretchable elastic member 15 is disposed between two thermoplastic nonwoven fabrics. The flap member 14 is folded in half along a fold line 14r between side edges 14n and 14m of the two nonwoven fabrics opposing each other, and extends in a around-torso direction 10x illustrated with an arrow 10x. In the flap member 14, a cutout 14k corresponding to a leg opening is formed. The elastic member 15 is, for example, a thread rubber or the like, and extends in the around-torso direction 10x. Fig. 1 illustrates a state where the elastic member 15 is stretched.

To the center in the around-torso direction 10x of the flap member 14, the back part 12a of the absorptive main body 12 is joined. The flap member 14 thus forms a pair of flap parts 14u provided on the back part 12a of the absorptive main body 12, and extending to the both sides in the around-torso direction 10x. In this case, the opposing member 13 forming the opposing part is provided, for example, on the non-skin surface side of the front part 12c of the absorptive main body 12.

Although not illustrated in the drawings, the front part 12c of the absorptive main body 12 may be joined to the center in the around-torso direction 10x of the flap member 14 instead of the back part 12a of the absorptive main body 12. In this case, the pair of flap parts 14u are provided in the front part 12c of the absorptive main body 12, and extend to the both sides in the around-torso direction 10x. The opposing member 13 forming the opposing part is provided, for example, on the non-skin surface side of the back part 12a of the absorptive main body 12.

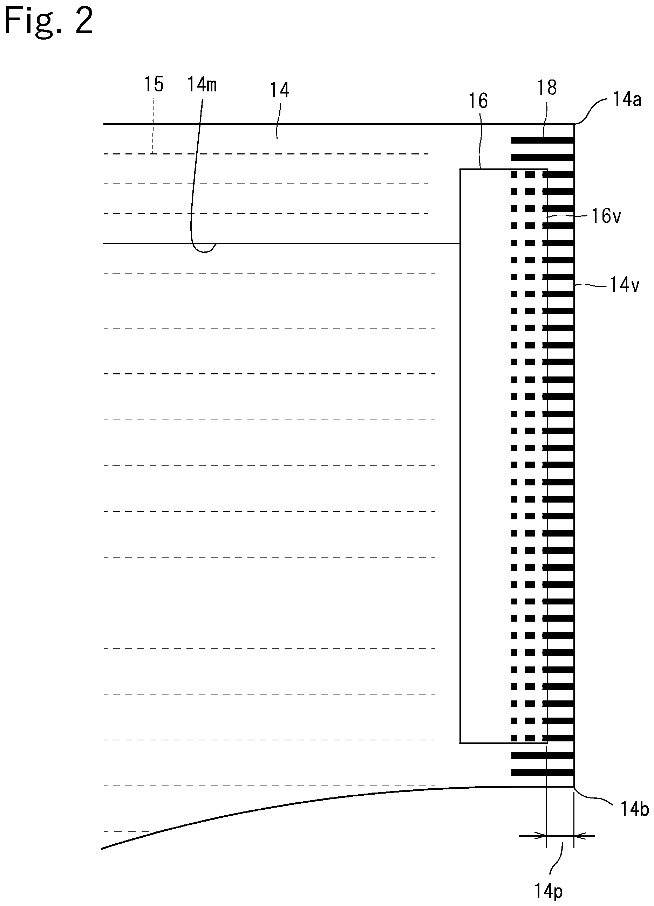

Fig. 2 is an enlarged view of an end portion in the around-torso direction 10x of the flap part 14u of the disposable wearable article 10. As illustrated in Figs. 1 and 2, in the flap part 14u, a fastener member 16 is disposed at a distance from the tip end 14v in the around-torso direction 10x of the flap member 14. The fastener member 16 is a hook-and-loop fastener, such as a hook fastener, to be attachably and detachably joined to the opposing member 13. In use of the disposable wearable article 10, the fastener member 16 is joined to the opposing member 13 so as to fasten the flap part 14u onto the front part 12c of the absorptive main body 12.

The flap part 14u has a thermally-fused part 18 having bending rigidity of the nonwoven fabric increased by thermally fusing the nonwoven fabric of the flap member 14. The thermally-fused part 18 is formed in an extending part 14p between the tip end 14v of the flap part 14u and the fastener member 16, and is further formed to extend beyond the extending part 14p continuously into a region of the flap part 14u where the fastener member 16 is disposed. The thermally-fused part 18 may be formed only in the extending part 14p.

The extending part 14p disposed between the tip end 14v of the flap member 14 and the fastener member 16 is made of a nonwoven fabric, and hence is lack of stiffness and easily folded as it is, and therefore, may be folded to be overlaid on the fastening surface of the fastener member 16, and remain to attach to the fastening surface of the fastener member 16 in some cases. When the fastener member 16 is fastened in this state, the fastening area is insufficient, which causes a problem of weak fastening power.

When the thermally-fused part 18 is formed in the extending part 14p, the rigidity of the extending part 14p is increased, and folding of the extending part 14p is suppressed. As a result, the extending part 14p disposed on the tip side of the flap part 14u is prevented from covering the fastening surface of the fastener member 16, and thus, sufficient fastening power of the fastener member 16 can be ensured.

The thermally-fused part 18 may be formed to be adjacent to only a part of a region between a first end 14a and a second 14b in a direction perpendicular to the around-torso direction of the tip end 14v of the flap part 14u, but is preferably formed to be adjacent to the whole region as illustrated in the drawings. When the thermally-fused part 18 is formed to be adjacent to the whole region between the first end 14a and the second end 14b, folding of the end on the tip side of the flap part 14u is suppressed in the whole region along the tip end 14V of the flap part 14u. As a result, sufficient fastening power of the fastener member 16 can be more definitely ensured.

When the thermally-fused part 18 is continuously provided from the extending part 14p into the region where the fastener member 16 is disposed as illustrated in Figs. 1 and 2, folding of the extending part 14p disposed on the tip side of the flap part 14u is suppressed from the base of the extending part 14p. As a result, sufficient fastening power of the fastener member 16 can be more definitely ensured.

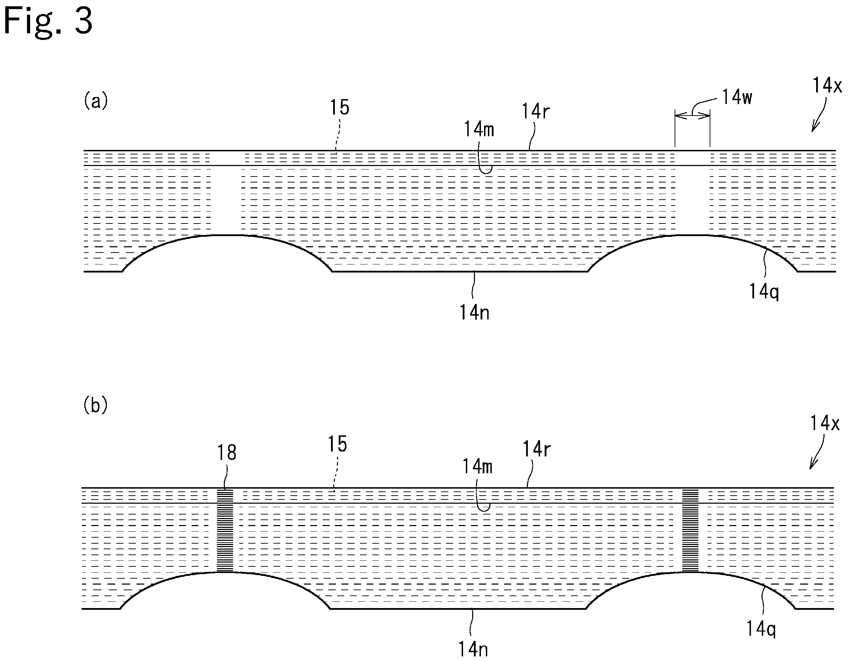

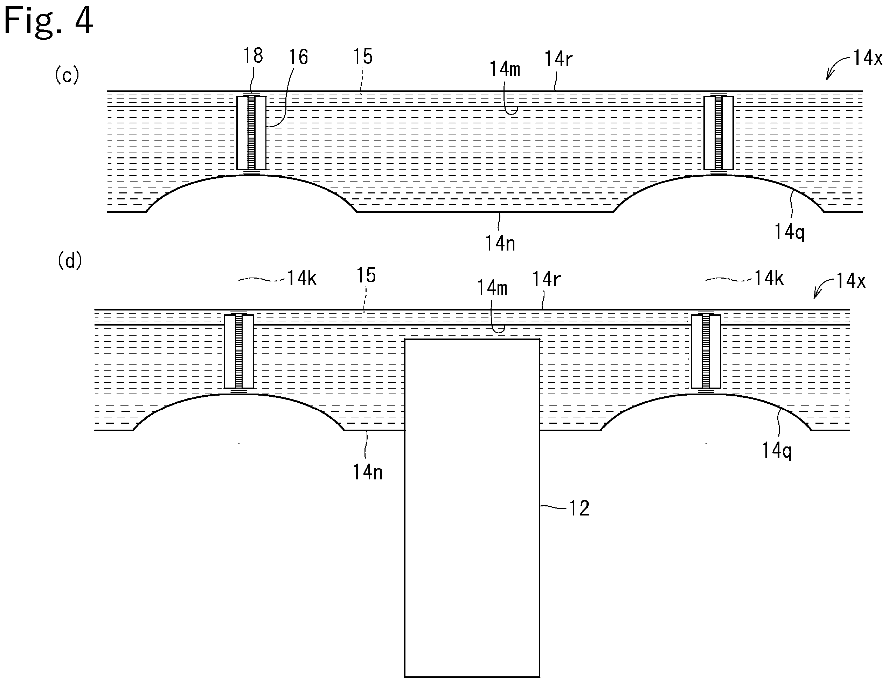

Next, processes for producing the disposable wearable article 10 will be described with reference to Figs. 3 to 5. Figs. 3 and 4 are explanatory diagrams of the processes for producing the disposable wearable article 10.

As illustrated in Fig. 3(a), a flap member continuous body 14x in which portions corresponding to the flap members 14 are connected to one another is prepared to be conveyed in the lengthwise direction. The flap member continuous body 14x is formed by disposing, between nonwoven fabric sheets, a continuous body of the elastic member 15 in a stretched state; mutually joining the nonwoven fabric sheets and the continuous body of the elastic member 15; and then cutting the continuous body of the elastic member 15 at appropriate positions. After the cutting, the elastic member 15 in a stretched state is not disposed in a tip end planned region 14w that is planned to correspond to the tip end of the flap part 14u.

Next, as illustrated in Fig. 3(b), when the flap member continuous body 14x under conveyance passes through a thermal fusion device, the center portion of the tip end planned region 14w is thermally fused to form a plurality of thermally-fused parts 18 extending linearly and in parallel to one another.

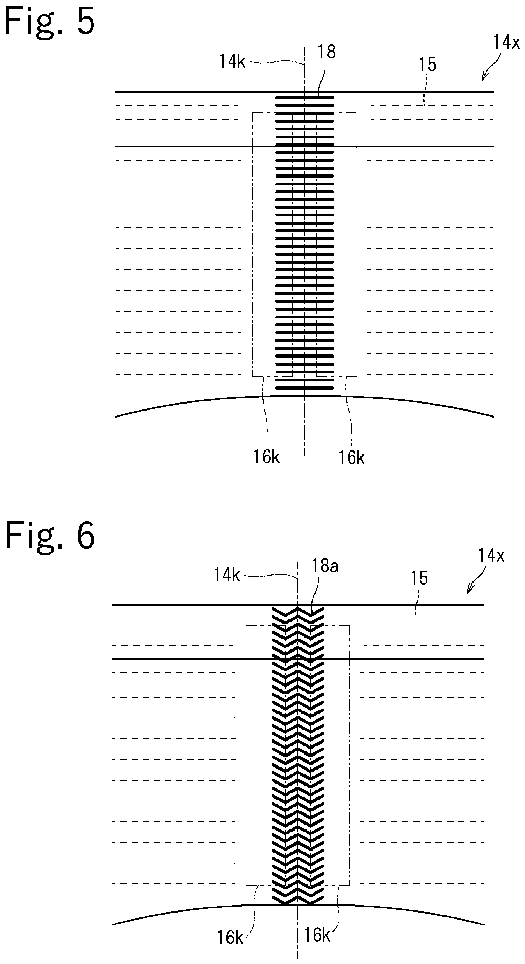

Fig. 5 is an enlarged view of the vicinity of the tip end planned region 14w. In Fig. 5, a dash-dot line 14k corresponds to a cut planned position 14k in which the flap member continuous body 14x is planned to be cut, and a dash-dot-dot line corresponds to a fastener member disposition region 16k in which the fastener member 16 is planned to be fixed. The plural thermally-fused parts 18 cross, at a right angle, the cut planned position 14k to be continuous into the fastener member disposition regions 16k on both sides of the cut planned position 14k. In a subsequent step, the flap member continuous body 14x is cut in the cut planned position 14k, thereby forming a plurality of thermally fused parts 18 linearly extending, and having a right angle to the tip end 14v of the flap part 14u.

The thermally-fused parts 18 can be easily formed when the flap member continuous body 14x conveyed in the lengthwise direction passes through a thermal fusion device, and therefore, the disposable wearable article 10 can be easily mass-produced.

Subsequently, as illustrated in Fig. 4(c), in the tip end planned region 14w of the flap member continuous body 14x, a pair of fastener members 16 are disposed on both sides of the cut planned position 14k at a distance from the cut planned position 14k, and are fixed by thermal fusion, adhesion or the like.

Subsequently, as illustrated in Fig. 4(d), the absorptive main body 12 is fixed on the flap member continuous body 14x by thermal fusion, adhesion or the like. Although not illustrated in the drawing, the opposing member 13 is precedently provided on the absorptive main body 12. Alternatively, after fixing the absorptive main body 12 on the flap member continuous body 14x, the opposing member 13 is provided on the absorptive main body 12. It is noted that the stretchability of the elastic member 15 may be eliminated or reduced by cutting the continuous body of the elastic member 15 in a region where the absorptive main body 12 is fixed.

Subsequently, the flap member continuous body 14x is cut in the cut planned position 14k illustrated with the dash-dot line 14k in Fig. 4(d) to form individuals of the disposable wearable article 10.

Through the processes described so far, the disposable wearable articles 10 can be successively formed.

Figs. 6 to 9 are explanatory diagrams of thermally-fused parts 18a to 18d of Modification Examples 1 to 4 of Example 1. Figs. 6 to 9 are enlarged views, similar to Fig. 5, of the vicinity of the tip end planned region 14w of the flap member continuous body 14x before cutting.

<Modification Example 1> The thermally-fused parts 18a of Modification Example 1 illustrated in Fig. 6 are formed in a zigzag shape, and diagonally cross (at a non-right angle) the cut planned position 14k and the fastener member disposition region 16k. The thermally-fused parts 18a linearly extend between the cut planned position 14k and the fastener member disposition region 16k (namely, a region corresponding to the extending part 14p). When the flap member continuous body 14x is cut in the cut planned position 14k, the thermally-fused parts 18a are formed in a V-shape in the flap part 14u, and linearly extend in the extending part 14p.

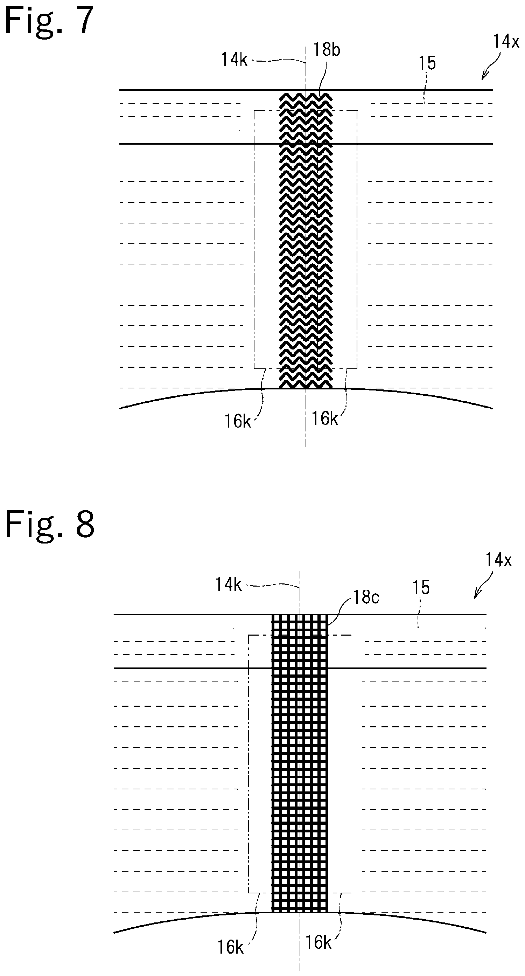

<Modification Example 2> The thermally-fused parts 18b of Modification Example 2 illustrated in Fig. 7 are formed in a further zigzag shape, and diagonally cross (at a non-right angle) the cut planned position 14k and the fastener member disposition region 16k. The thermally-fused parts 18b extend in a V-shape between the cut planned position 14k and the fastener member disposition region 16k (namely, a region corresponding to the extending part 14p). When the flap member continuous body 14x is cut in the cut planned position 14k, the thermally-fused parts 18b are formed in a W-shape in the flap part 14u, and extend in a V-shape in the extending part 14p.

<Modification Example 3> The thermally-fused parts 18c of Modification Example 3 illustrated in Fig. 8 are formed in a lattice shape, and include a plurality of first linear portions extending in parallel to the cut planned position 14k, and a plurality of second linear portions crossing, at a right angle, the cut planned position 14k. The thermally-fused parts 18c in a lattice shape suppresses folding in all directions of the end on the tip side of the flap part 14u. As a result, sufficient fastening power of the fastener member 16 can be more definitely ensured.

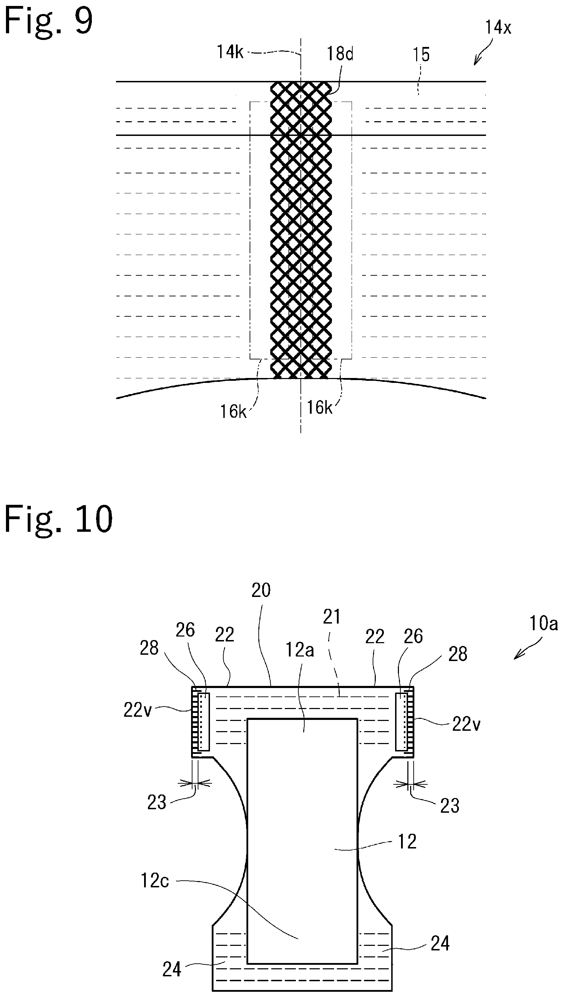

<Modification Example 4> The thermally-fused parts 18d of Modification Example 4 illustrated in Fig. 9 are formed in a diagonal lattice shape, and include a plurality of first and second linear portions diagonally crossing the cut planned position 14k and parallel to one another. Although the first and second linear portions cross each other at a right angle in the drawing, the first and second linear portions may be diagonally (at a non-right angle) cross each other. The thermally-fused parts 18d in a lattice shape suppresses folding in all directions of the end on the tip side of the flap part 14u. As a result, sufficient fastening power of the fastener member 16 can be more definitely ensured.

<Example 2> A disposable wearable particle 10a of Example 2 will now be described with reference to Fig. 10. Fig. 10 is a plan view of the disposable wearable article 10a.

As illustrated in Fig. 10, in the disposable wearable article 10a, an adsorptive body 12 similar to that of Example 1 is joined to a flap member 20 in which a stretchable elastic member 21 is disposed between two thermoplastic nonwoven fabrics. Owing to the flap member 20, a pair of first flap parts 22 extending to both sides in the around-torso direction are provided in a back part 12a of the absorptive main body 12, and a pair of second flap parts 24 extending to the both sides in the around-torso direction are provided in a front part 12c of the absorptive main body 12.

In the first flap parts 22, a pair of fastener members 26 are respectively provided at a distance from tip ends 22v in the around-torso direction of the first flap parts 22. The fastener members 26 are attachably and detachably joined to the second flap parts 24. The second flap parts 24 are opposing parts provided on the absorptive main body 12. In the second flap parts 24, opposing members of a loop fastener or the like to be joined to the fastener members 26 of a hook fastener or the like may be provided. Each of the first flap parts 22 is made of a thermoplastic nonwoven fabric, and has, in an extending part 23 between the tip end 22v of the first flap part 22 and the fastener member 26, a thermally-fused part 28 having bending rigidity of the nonwoven fabric increased by thermally fusing the nonwoven fabric. The thermally-fused part 28 extends, beyond the extending part 23, continuously into a region where the fastener member 26 of the first flap part 22 is disposed. The thermally-fused part 28 may be formed only in the extending part 23.

In the disposable wearable article 10a, the thermally-fused part 28 can suppress folding of the portion of the first flap part 22 protruding to the end on the tip side in the around-torso direction beyond the fastener member 26, namely, the extending part 23, and prevents the first flap part 22 from covering the fastener member 26, and thus, sufficient fastening power of the fastener member 26 can be ensured.

In the disposable wearable article 10a of Example 2, thermally-fused parts in a zigzag shape or lattice shape may be formed in the same manner as in Modification Examples 1 to 4 described above.

<Conclusion> As described so far, since the flap part has the thermally-fused part, the folding of the portion of the flap part protruding to the end on the tip side in the around-torso direction beyond the fastener member, namely, the extending part, can be suppressed, and the flap part is prevented from covering the fastener member, and thus, sufficient fastening power of the fastener member can be ensured.

It is noted that the present invention is not limited to the above-described embodiments, but can be practiced with various changes and modifications.

For example, the thermally-fused part may be formed in a curved shape, or in a staggered shape. The thermally-fused part may be formed at a distance from the tip end 14v of the flap part 14u, or may be formed at a distance from the region where the fastener member 16 is disposed. Besides, the configurations described in Example 1, Modification Examples 1 to 4, and Example 2 may be appropriately combined.

### DESCRIPTION OF REFERENCE NUMERALS

10, 10a disposable wearable article
10x around-torso direction
12 absorptive main body
12a back part
12b crotch part
12c front part
13 opposing member (opposing part provided in absorptive main body)
14a first end
14b second end
14p extending part
14u flap part
14v tip end
16 fastener member
18, 18a to 18d thermally-fused part
22 first flap part
22v tip end
23 extending part
24 second flap part (opposing part provided in absorptive main body)
26 fastener member
28 thermally-fused part

## Claims

1. A disposable wearable article (10, 10a), comprising:
an absorptive main body (12) in which an absorber is disposed between a top sheet and a back sheet;
a pair of flap parts (14u, 22) that are provided in one of a front part (12c) and a back part (12a) disposed on both side of a crotch part (12b) of the absorptive main body (12), and extend to both sides in a around-torso direction (10x); and
a pair of fastener members (16, 26) that are respectively provided in the pair of flap parts (14u, 22) at a distance from tip ends (14v, 22v) in the around-torso direction (10x) of the flap parts (14u, 22) to be attachably and detachably joined to an opposing part provided in the absorptive main body (12),
wherein each of the flap parts (14u, 22) is made of a thermoplastic nonwoven fabric, and includes, in an extending part (14p, 23) between the tip end (14v, 22) of the flap part (14u, 22) and the fastener member (16, 26), a thermally-fused part (18, 18a to 18d, 28) having bending rigidity of the nonwoven fabric increased by thermally fusing the nonwoven fabric.

2. The disposable wearable article (10, 10a) according to claim 1, wherein the thermally-fused part (18, 18a to 18d, 28) continuously extends from the extending part (14p, 23) into a region of the flap part (14u, 22) where the fastener member (16, 26) is disposed.

3. The disposable wearable article (10, 10a) according to claim 1 or 2, wherein each of the flap parts (14u, 22) includes a plurality of the thermally-fused parts (18, 28) linearly extending at a right angle to the tip end (14v, 22v) of the flap part (14u, 22).

4. The disposable wearable article (10, 10a) according to any one of claims 1 to 3, wherein each of the flap parts (14u, 22) includes the thermally-fused part (18c, 18d) formed in a lattice shape.

5. The disposable wearable article (10, 10a) according to any one of claims 1 to 4, wherein each of the flap parts (14u, 22) includes the thermally-fused part (18, 18a to 18d, 28) formed adjacently to a whole region between a first end (14a) and a second end (14b), in a direction crossing the around-torso direction (10x) at a right angle, of the tip end (14v, 22v) of the flap part (14u, 22).

## Patentansprüche

1. Ein tragbarer Einwegartikel (10, 10a)einschließend:
ein absorbierender Hauptkörper (12) bei dem ein Absorber zwischen einem Deckblech und einem Rückblech angeordnet ist;
ein Paar Klappenteile (14u, 22) die in einem der vorderen Teile vorgesehen sind (12c) und ein Rückenteil (12a) beidseits eines Schrittteils angeordnet (12b) des absorbierenden Hauptkörpers (12)und erstrecken sich zu beiden Seiten in Richtung des Oberkörpers (10x); und
ein Paar Befestigungselemente (16, 26) die jeweils in dem Paar Klappenteile vorgesehen sind (14u, 22) in einiger Entfernung von den Spitzenenden (14v, 22v) in Um-Rumpf-Richtung (10x) der Klappenteile (14u, 22) aufsteckbar und lösbar mit einem gegenüberliegenden Teil verbunden zu sein, das in dem absorbierenden Hauptkörper vorgesehen ist (12),
wobei jedes der Klappenteile (14u, 22) besteht aus einem thermoplastischen Vliesstoff und umfasst in einem ausziehbaren Teil (14p, 23) zwischen dem Spitzenende (14v, 22) des Klappenteils (14u, 22) und das Befestigungselement (16, 26), ein thermisch verschmolzenes Bauteil (18, 18a bis 18d, 28) Erhöhung der Biegesteifigkeit des Vliesstoffs durch thermisches Verschmelzen des Vliesstoffs.

2. Der tragbare Einwegartikel (10, 10a) nach Anspruch 1, wobei das thermisch verschmolzene Teil (18, 18a bis 18d, 28) Erstreckt sich kontinuierlich vom ausziehbaren Teil (14p, 23) in einen Bereich des Klappenteils (14u, 22) wo das Verbindungselement (16, 26) verfügt wird.

3. Der tragbare Einwegartikel (10, 10a) nach Anspruch 1 oder 2, wobei jedes der Klappenteile (14u, 22) Enthält eine Vielzahl von die thermisch verschmolzenen Teile (18, 28) linear im rechten Winkel zum Spitzenende verlängerbar (14v, 22v) des Klappenteils (14u, 22).

4. Der tragbare Einwegartikel (10, 10a) nach einem der Ansprüche 1 bis 3, wobei jedes der Klappenteile (14u, 22) Enthält das thermisch abgesicherte Teil (18c, 18d) gitterförmig geformt.

5. Der tragbare Einwegartikel (10, 10a) nach einem der Ansprüche 1 bis 4, wobei jedes der Klappenteile (14u, 22) Enthält das thermisch abgesicherte Teil (18, 18a bis 18d, 28) angrenzend an eine ganze Region zwischen einem ersten Ende (14a) und ein zweites Ende (14b), in einer Richtung, die die Um-Rumpf-Richtung kreuzt (10x) im rechten Winkel des Spitzenendes (14v, 22v) des Klappenteils (14u, 22).

## Revendications

1. Un article portable jetable (10, 10a)Comprenant:
un corps principal absorbant (12) dans lequel un absorbeur est disposé entre une feuille supérieure et une feuille arrière ;
une paire de pièces de rabat (14u, 22) qui sont prévus dans l'une des parties avant (12c) et une partie arrière (12a) disposé des deux côtés d'une partie de l'entrejambe (12b) du corps principal absorbant (12), et s'étendent des deux côtés dans une direction autour du torse (10x); et
une paire d'éléments de fixation (16, 26) qui sont respectivement prévus dans la paire de parties du volet (14u, 22) à une distance des extrémités des pointes (14v, 22v) dans le sens du tour du torse (10x) des pièces du rabat (14u, 22) à joindre de manière attachante et détachable à une partie opposée prévue dans le corps principal absorbant (12),
dans laquelle chacune des parties du rabat (14u, 22) est fait d'un tissu non tissé thermoplastique, et comprend, dans une partie extensible (14p, 23) entre l'extrémité de la pointe (14v, 22) de la partie volet (14u, 22) et l'élément de fixation (16, 26), une pièce thermofusionnée (18, 18a à 18d, 28) La rigidité à la flexion du tissu non tissé a été augmentée par fusion thermique du tissu non tissé.

2. L'article portable jetable (10, 10a) selon la revendication 1, dans laquelle la partie thermofusionnée (18, 18a à 18d, 28) s'étend en continu à partir de la partie extensible (14p, 23) dans une région de la partie du lambeau (14u, 22) où l'élément de fixation (16, 26) est éliminé.

3. L'article portable jetable (10, 10a) selon la revendication 1 ou 2, dans laquelle chacune des parties du rabat (14u, 22) Comprend une pluralité de Les pièces thermofusionnées (18, 28) s'étendant linéairement à angle droit par rapport à l'extrémité de la pointe (14v, 22v) de la partie volet (14u, 22).

4. L'article portable jetable (10, 10a) selon l'une quelconque des revendications 1 à 3, dans laquelle chacune des parties du volet (14u, 22) comprend la partie thermofusionnée (18c, 18d) en forme de treillis.

5. L'article portable jetable (10, 10a) selon l'une quelconque des revendications 1 à 4, dans laquelle chacune des parties du volet (14u, 22) comprend la partie thermofusionnée (18, 18a à 18d, 28) formé à côté d'une région entière entre une première extrémité (14a) et une seconde fin (14b), dans une direction croisant la direction autour du torse (10x) à angle droit, de l'extrémité de la pointe (14v, 22v) de la partie volet (14u, 22).
